# NEW EUROPEAN PATENT SPECIFICATION

(11) **EP 2 869 707 B2**
(45) Date of publication and mention of the opposition decision: **28.09.2022**
(45) Mention of the grant of the patent: 09.08.2017
(21) Application number: 13760131.6
(22) Date of filing: 09.07.2013
(51) Int. Cl.: A23D 9/00, A61K 38/01, A61K 38/17, A61K 47/46, A61K 36/48, A23L 33/18, A23L 33/185, A23L 33/19, A23J 3/14, A61K 38/16, A61K 9/00, A61K 35/20, A23L 33/00

(54) **METHOD FOR PRODUCING A PROTEIN AND LIPID COMPRISING COMPOSITION WITH REDUCED DIGESTIVE COAGULATION**
VERFAHREN ZUR HERSTELLUNG EINES PROTEINS UND EINES LIPIDS MIT EINER ZUSAMMENSETZUNG MIT VERRINGERTER VERDAUUNGSGERINNUNG
PROCÉDÉ DE PRODUCTION D'UNE COMPOSITION COMPRENANT DES PROTÉINES ET DES LIPIDES AVEC UNE COAGULATION DIGESTIVE RÉDUITE

(30) Priority: 09.07.2012 WO PCT/NL2012/050489
(43) Date of publication of application: 13.05.2015
(73) Proprietor: N.V. Nutricia, 2712 HM Zoetermeer (NL)
(72) Inventor: VAN DEN BRAAK, Claudia Catharina Maria, NL-3584 CT Utrecht (NL); LUDWIG, Thomas, NL-3584 CT Utrecht (NL); MINOR, Marcel, NL-6704 AA Wageningen (NL); VERDURMEN, Rudolph Eduardus Maria, NL-3584 CT Utrecht (NL); RUIS, Hilde, NL-3584 CT Utrecht (NL)
(74) Representative: Nederlandsch Octrooibureau
(86) International application number: PCT/NL2013/050520
(87) International publication number: WO 2014/011040

(56) References cited:
- EP-A1- 1 314 361
- EP-A1- 1 762 147
- WO-A1-2006/029298
- WO-A1-2009/046386
- WO-A1-2010/042932
- WO-A1-2010/126362
- WO-A1-2011/093693
- US-A1- 2003 104 033
- BELICIU C. M., MORARU C. I.: "The Effect of Protein Concentration and Heat Treatment Temperature on Micellar Casein - Soy protein Mixtures", FOOD HYDROCOLLOIDS, vol. 25, 2011, - 2011, pages 1448-1460,
- ALZATE A. B.: "Pasteurization of Milk", UNDERGRADUATE JOURNAL OF MATHEMATICAL MODELLING: ONE + TWO, vol. 2, no. 2, 2010, - 2010,

## Description

### Field of the invention

This invention is in the field of protein-containing nutritional compositions. In particular, this invention concerns the coagulation of such compositions in the upper gastrointestinal tract, more in particular in the stomach. This invention aims to modulate the digestive coagulation of proteins and peptic digestion, by adjusting the production process of nutritional compositions comprising proteins and lipids.

### Background of the invention

Coagulation of proteins in the upper gastro-intestinal tract, in particular in the stomach, is hypothesised to delay gastric emptying. This can result in upper gastro-intestinal complications like reflux, gastrointestinal discomfort, aspiration pneumonia, but also to confer satiety and the feeling of having a full stomach when this is not intended yet. Nutritional compositions that mainly contain caseins are desired for their nutritional value, but these compositions in particular tend to coagulate under the acidic conditions encountered in the stomach.

One manner of providing digestion support to subjects in need thereof is to administer nutrition that results in lower coagulation levels in the stomach. The possibility to reduce digestive coagulation is preferred for those subjects suffering from upper gastrointestinal-related conditions such as intestinal discomfort, reflux, aspiration pneumonia, high gastric residual volume (GRV), vomiting, nausea, bloating, and delayed gastric emptying. Further, facilitating digestibility is desired when aiming to promote digestive comfort, reduce gastrointestinal cramping or colics. On the other hand, if slower release of stomach contents, slow absorption of nutrients, a certain level of fullness perception or satiety is intended, a certain level of coagulation of nutritional compositions within the stomach can be desirable. Therefore, having the ability to influence gastric coagulation levels can be desirable.

Nutritional compositions containing casein, in particular sodium caseinate, vegetable proteins, such as soy and/or pea protein, and a lipid source are known.

For example US 2003/0104033 teaches enteral formulations comprising 40-95 weight% of caseinate and 5 -60 weight% of a stabilising protein, selected from the group of whey protein and one or more vegetable proteins selected from the group of soy, corn, potato, rice and pea, the most preferred vegetable protein being soy protein. The document is concerned with the reduction of creaming in enteral formulae and is silent with respect to coagulation properties of the composition.

Another example is EP 1 972 346 (WO2007/063142) which discloses a pea-based protein mixture comprising 50 weight% caseinate, 25 weight% whey proteins and 25 weight% pea protein. The document is silent with respect to coagulation properties of the composition.

Another example is WO2010/131952, wherein a method for reducing digestive coagulation of proteins is disclosed. Herein it is described that combining casein and anti-coagulating proteins in nutritional compositions reduces gastric coagulation effects. Besides the measures mentioned therein, the document makes no suggestions on how to further reduce gastric coagulation of coagulation proteins in a nutritional composition comprising a lipid source.

Another example is EP1314361 wherein the whey protein and the NA-caseinate are separately UHT-sterilised prior to mixing and aseptic filling.

### Summary of the invention

The present inventors found that gastric coagulation of a given protein composition that contains a casein and a lipid can be modulated by adjusting the production process of the nutritional composition. For instance, it was observed that reduction in coagulation was much more than was expected based on the ratio of casein and anti-coagulating proteins that were included, together with a lipid, in the composition. Thus by separately heat-sterilising a casein and an anti-coagulating protein, digestive coagulating of the protein fraction of a nutritional composition further comprising a lipid can be influenced to achieve desirably low coagulation levels.

The present invention therefore relates to a process of producing a composition, preferably a nutritional composition, comprising at least one lipid and a mixture of two different proteins, of which at least one is a casein and at least one is an anti-coagulating protein, comprising the steps of:
a) heat-sterilising a first liquid component which comprises said casein in an amount of at least 85 wt% of the total protein content of the first component, and wherein said first liquid component comprises less than 5 wt% anti-coagulating protein based on the total protein content of the first liquid component, and
b) heat-sterilising a second liquid component comprising said anti-coagulating protein, wherein said anti-coagulating protein is selected from pea protein and soy protein; and wherein said second liquid component comprises less than 0.1 wt% of casein based on the total protein content of the second liquid component, and
c) mixing said first component with said second component to obtain a mixture of said at least two different proteins, wherein said first and/or said second liquid component comprises said lipid, and wherein said mixture has a weight ratio of said casein to said anti-coagulating protein of between 10:1 and 1:1.

Preferably, said mixture comprises an effective amount of anti-coagulating protein such that coagulation of said casein is reduced in the stomach of a subject. To this end, said mixture has a weight ratio of said casein to said anti-coagulating protein of any value between 10:1 and 1:1. Preferably, this mixture is part of a nutritional composition further comprising said lipid.

Preferably, at least said second liquid component comprises said lipid, preferably during heat-sterilisation.

Furthermore, the invention relates to a process of producing a composition comprising a lipid and a mixture of casein and anti-coagulating proteins, in a weight ratio of between 10:1 and 1:1, for reducing or preferably preventing coagulation in the upper gastrointestinal tract.

The process of the invention includes step a) which involves heat-sterilising said first liquid component which comprises said casein in an amount of at least 85 wt% of the total protein content of the first component, and wherein said first liquid component comprises less than 5 wt% anti-coagulating protein based on the total protein content of the first liquid component, and includes step b) which involves heat-sterilising said second liquid component which comprises said anti-coagulating protein, selected from pea protein and soy protein, wherein said second liquid component comprises less than 0.1 wt% of casein based on the total protein content of the second liquid component, and followed by mixing of the two components, wherein the first and/or second liquid component comprises lipid, such that a composition is obtained which comprises lipid and a mixture of casein and anti-coagulating proteins in a weight ratio which is between 10:1 and 1:1.

Preferred caseins used in the process of the invention comprise (or preferably consist of) micellar casein and/or caseinate proteins. The process of the present invention can be performed such that a composition is obtained which comprises a mixture of caseins, preferably micellar casein and/or caseinate, and pea or soy protein or a combination thereof, having a weight ratio of casein to anti-coagulating proteins ranging between 10:1 and 1:1.

Preferred lipids (such as oils and fats), include compounds containing fatty acids, such as free fatty acids, esters, monoglycerides, diglycerides, triglycerides, and phospholipids as defined below. The fatty acids comprise saturated fatty acids, mono-unsaturated fatty acids and poly-unsaturated fatty acids. preferably including α-linolenic acid. The lipids may comprise fish oil, microbial (including algal) oil, vegetable oil and/or animal fats, more preferably fish oil and/or vegetable oil such as palm oil, canola oil and sunflower oil. Preferred fish oils include long-chain omega-3 fatty acids, such as eicosapentaenoic acid (EPA), docosahexaenoic acid (DHA).

Furthermore, the process of the invention may further comprise a drying step to obtain a dry composition, e.g. in the form of a powder. If a drying step is desired, it may be included after heat-sterilising the first and/or second liquid components of steps a) and b) but before the mixing thereof in step c), such that mixing is performed with at least the dried heat-sterilised first component and the dried heat-sterilised second component, wherein the first and/or second component comprises a lipid. Alternatively, such a drying step is performed after the heat-sterilised first liquid component is mixed with the heat-sterilised second liquid component in step c), such that mixing in step c) is performed with a liquid mixture of heat-sterilised casein and anti-coagulating proteins and lipids which is subsequently dried, for instance to obtain a powder.

Preferably, step c) as mentioned herein comprises the mixing of a heat-sterilised first liquid component with a heat-sterilised second liquid component, wherein either or both comprises a lipid, to obtain a mixture of lipids and casein and anti-coagulating proteins in liquid form. It is preferred that the second liquid component comprises the lipid.

Another aspect of the invention relates to a composition obtainable by the process of the present invention. Said composition comprises a lipid and at least two different proteins, of which at least one is a casein, preferably comprising or consisting of a casein, and of which at least one is an anti-coagulating protein, selected from pea, soy or a combination thereof. Said composition preferably comprises a lipid and a mixture of casein to anti-coagulating proteins having a weight ratio which ranges between 10:1 and 1:1.

The process of the invention is performed such that casein and anti-coagulating protein are separately heat-sterilised, *i.e.* without having been mixed with each other before the heat-sterilisation steps. Surprisingly, the composition obtainable by the process of the invention which comprises a lipid and said mixture of casein and anti-coagulating proteins is characterised by having reduced coagulation properties when ingested.

Preferably, said mixture of anti-coagulating and caseins according to the present invention constitutes, in liquid form, between 1 and 20% (w/v), preferably between 2 and 15% (w/v), more preferably between 4 and 12% (w/v), most preferably between 5 and 10% (w/v) of the composition. Preferably said composition is a liquid nutritional composition suitable for enteral feeding.

In a preferred embodiment, said lipid comprises between 1 and 30 wt% of the composition obtainable according to the invention, more preferably between 3 and 20 wt%, most preferably between 4 and 15 wt%.

Consumption of the composition serves to lower coagulate particle size and/or reduce the number of coagulate particles in the stomach of said person. This is expected to improve peptic digestion and gastric emptying of the stomach content of said person. Thus, the invention further relates to the non-therapeutic use of a composition obtainable by the process according to the present invention in the reduction of coagulation in the upper gastrointestinal tract, in particular the stomach, of the person.

Again another aspect of the present invention is concerned with a method of providing relief of gastric problems by administering or providing the composition obtainable by the process according to the present invention to a person. Consumption of the composition serves to lower coagulate particle size and/or reduce the number of coagulate particles in the stomach of said person or patient. This is expected to improve peptic digestion and gastric emptying of the stomach content of said person or patient. Thus, the invention further relates to the use of a composition obtainable by the process according to the present invention in the reduction or preferably prevention of coagulation in the upper gastrointestinal tract, in particular the stomach, of the person or patient.

### Detailed description of the invention

### Process according to the invention

This invention aims to reduce the digestive coagulation of proteins through the provision of an improved production process of providing compositions comprising lipid and protein; preferably such a composition is a nutritional composition.

The present invention therefore relates to a process of producing a composition comprising at least one lipid and a mixture of two different proteins, of which at least one is a casein and at least one is an anti-coagulating protein, comprising the steps of:
a) heat-sterilising a first liquid component which comprises said casein in an amount of at least 85wt%, preferably a least 90 wt%, more preferably at least 95 wt% of the total protein content of the first component, and wherein said first liquid component comprises less than 5 wt% anti-coagulating protein based on the total protein content of the first liquid component, and
b) heat-sterilising a second liquid component comprising said anti-coagulating protein, wherein said anti-coagulating protein is selected from pea and soy protein; wherein said second liquid component comprises less than 0.1 wt% of casein based on the total protein content of the second liquid component, and
c) mixing said first component with said second component to obtain a mixture of said at least two different proteins, wherein said first and/or said second liquid component comprises said lipid, and wherein said mixture has a weight ratio of said casein to said anti-coagulating protein of between 10:1 and 1:1.

Preferably, said mixture of casein and anti-coagulating proteins and said lipid is part of a nutritional composition. Preferably, the casein is present in an amount of at least 50%, most preferably between 60 and 90 wt% of the total protein of the composition obtainable by the method of the invention.

The mixing according to step c) of casein with anti-coagulating protein results in the provision of a mixture of casein (preferably micellar casein and/or caseinate) and anti-coagulating pea or soy protein or a combination thereof, having a weight ratio of casein to anti-coagulating protein which is between 10:1 and 1:1.

It is to be understood that, within the context of the present invention, said heat-sterilisation steps a) and b) of the process of the invention are performed such that the first liquid component and the second liquid component are not mixed with each other during heating steps a) and b). To ensure that these components are kept separated as intended, said first and second liquid components are typically heat-sterilised in separate holders, containers or any other suitable means.

Said first liquid component comprises less than 5 wt% based on the total protein content of the first component. Said anti-coagulating protein comprises or consist of pea, soy and/or a combination thereof.

The second liquid component is substantially free of casein. Within the context of the present invention the phrase "substantially free of" is meant to be understood as that the second liquid component of step b) comprise only a small amount of casein, less than 0.1% relative to the total amount of protein present in the second liquid component.

It is furthermore preferred that the second liquid component is substantially free of casein. The heat-sterilised liquid components used in steps a) and b) are preferably aqueous solutions, emulsions or dispersions containing said proteins in a concentration of between 0,1 and 20 wt% (w/v) (*i.e.* 1-200 g/l), preferably 1-20 wt%, more preferably 2-15 wt%, most preferably 4-12 wt% or even 5-10 wt%. Preferably, the pH thereof is in the range of 5-9, more preferably 6-8.

The amounts of anti-coagulating and caseins used in steps a) and b) are selected such that the resulting mixture is characterised by a weight ratio of casein to anti-coagulating of between 10:1 to 1:1.

In one embodiment, the amount of casein in the first liquid component is between 0.5 and 19 wt%, preferably 1-14 wt%, more preferably 2-11 wt%, most preferably 3-9 wt%, whereas the amount of anti-coagulating protein in the second liquid component is between 0.05-10wt%, preferably 0.1-8 wt%, more preferably 0.2-6 wt%, most preferably 0.3-5 wt%. Other values can be calculated on the basis of the preferred mixture of casein and anti-coagulating proteins and the amount thereof as used in the composition.

As a consequence of the separate heat-sterilising of said casein and anti-coagulating proteins in steps a) and b) of the process of the invention, mixing step c) is performed with heat-sterilised casein and anti-coagulating proteins which have not been in contact with each other prior to said mixing in the process of the invention. Said mixing in step c) preferably involves mixing of two liquid components comprising at least one lipid and said mixture of casein and anti-coagulating proteins. Preferably, the pH of the resulting mixed liquid is in the range of 5-9, more preferably 6-8. In case a drying step is included in the process of the invention before said mixing in step c) takes place, said mixing in step c) preferably comprises mixing of dry first and second components to obtain a dry composition, such as a powder. In another embodiment the dry first component comprising the casein is mixed with said liquid second component comprising said anti-coagulating protein, or a liquid first component is mixed with a dry second component. Said lipid is included in the first and/or the second liquid component, preferably at least or only in said second liquid component.

Preferred lipids (such as oils and fats), include compounds containing fatty acids, such as free fatty acids, their esters, monoglycerides, diglycerides, triglycerides, and phosphatides of mono-and diglycerides (phospholipids). Preferably at least 50 wt%, more preferably at least 80 wt.%, of the lipids comprises triglycerides. The fatty acids comprise saturated fatty acids, mono-unsaturated fatty acids and poly-unsaturated fatty acids. The lipids may comprise fish oil (or microbial, including algal oil), vegetable oil and/or animal fats, more preferably fish oil and/or vegetable oil such as palm oil, canola oil and sunflower oil. Preferred fish oils include long-chain omega-3 fatty acids, such as eicosapentaenoic acid (EPA), docosahexaenoic acid (DHA). Preferably, the lipids comprise between 5 and 35 wt.% of polyunsaturated fatty acids, comprising between 0.5 and 10 wt.% of alpha-linolenic acid (ALA) on total fatty acid basis.

In a preferred embodiment, said lipid comprises between 1 and 30 wt% (w/v) of the composition in liquid form obtainable according to the invention, more preferably between 3 and 20 wt%, most preferably between 4 and 15 wt%.

In the same or another embodiment, the first liquid component contains between 0 and 80 wt.%, preferably between 0 and 30 wt.%, more preferably between 0 and 10 wt.% of lipids on dry matter basis, in particular it is substantially free of lipids. In the same or a further embodiment, the second component contains between 10 and 90 wt.%, preferably between 20 and 80 wt.%, more preferably between 30 and 75 wt.% of lipids on dry matter basis. Preferably, the lipid to protein weight ratio in the first liquid component is between 0 and 4, preferably between 0 and 1, most preferably below 0.2. Preferably, the lipid to protein weight ratio in the second liquid component is between 0.4 and 5, preferably between 0.6 and 4, most preferably between 1 and 3.

In the process of the present invention, heat-sterilisation of the first and second liquid components in step a) and b) is performed with any suitable method, such as retort sterilisation, ultra-high temperature (UHT) treatment or direct-steam injection (DSI). Heat-sterilisation comprises heating to a temperature of at least 100°C, most preferably at least 110°C. Said temperature is applied for a sufficient period to accomplish sterilisation,

Heat-sterilisation of the first and/or second liquid components in step a) and/or b) of the process of the present invention can be preceded or followed by a homogenisation step. Homogenisation of said liquid components may be accomplished by the skilled person using methods known to him, such as shaking, stirring, inverting or vortexing of the liquid components at a suitable temperature for a suitable period of time such that a homogenised liquid is obtained. Thus, in a preferred embodiment of the invention, the heat-sterilisation of said first and/or second liquid components comprises heat-sterilisation of homogenised first and/or homogenised second liquid components, which are subsequently mixed in step c). In an alternative embodiment, the first and/or second liquid components are first heat-sterilised according to step a) and/or b), followed by a homogenising step of said liquid components, after which step c) is performed. In another alternative embodiment, the first and/or second liquid components are first heat-sterilised according to step a) and/or b), followed by mixing according to step c), after which the composition is subjected to a homogenising step of said liquid components.

In a further embodiment of the process of the invention, at least one drying step is included. Preferably, such a drying step takes place before the mixing in step c), such that the heated first liquid component and the heated second liquid component are dried, e.g. to obtain a powder, which components are subsequently mixed or dry blended. In this embodiment, step c) comprises mixing of dry or dried first and second component to obtain a dry mixture thereof. This powder is thus a mixture of at least one lipid and separately heat-sterilised and dried casein and anti-coagulating proteins, which mixture has a dry weight ratio of said casein to said anti-coagulating protein of between 10:1 and 1:1.

Alternatively, the drying step of the process of the invention is included after the mixing in step c), wherein said mixture of at least one lipid and first and second liquid components form is dried, e.g. to obtain a powder. This powder is thus a dried mixture of said lipid and separately heat-sterilised casein and anti-coagulating proteins which mixture has a dry weight ratio of said casein to said anti-coagulating protein of between 10:1 and 1:1. As a further alternative a pre-drying step resulting in partially dried mixtures may precede mixing step c), and a post-drying step may follow resulting in a powder.

In a further embodiment of the process of the invention, the mixing in step c) is performed under sterile conditions. In a preferred embodiment, the process of the invention further comprises a step d), subsequent to step c), wherein the liquid product is aseptically filled into a holder. Such aseptically filling allows the liquid product to retain its shelf-life for a suitable time period, such as at least 9 months.

In a preferred embodiment of the invention, further food components are added prior to, during or after performing any of the steps a), b) and/or c). Such further food components can be added to the heat-sterilised first and/or heat-sterilised second liquid components and subsequently mixed or added after the mixing of first and second components in step c), preferably followed by a homogenisation step. Preferably, said further food components are added in food-grade quality (meaning they are sterilised, pasteurised or filtrated) which obviates having to subject the composition to any further processing steps that could reduce the quality, shelf-life or stability *et cetera* of the composition.

The further food components typically comprise conventional food ingredients or constituents, such as digestible carbohydrates, fibres, vitamins, minerals, single amino acids, dipeptides, tripeptides and oligopeptides. In a preferred embodiment, the further food components are selected from at least 1 wt%, preferably at least 4 wt%, up to e.g. 20 wt% of digestible carbohydrates and at least 0.1 wt%, preferably at least 0.5 wt.% of dietary fibres, up to e.g. 3 wt% based on the total weight of the first and/or second liquid component. Alternatively, the further food components are selected from at least 1 wt%, preferably at least 10 wt%, up to e.g. 250 wt% of digestible carbohydrates and at least 0.1 wt%, preferably at least 1 wt.%, up to e.g. 25 wt% of dietary fibres, relative to the total weight of the mixture of proteins as obtained after step c).

In another embodiment of the invention, a further protein, such as defined herein below, is added to the process of the invention. Preferably, the amount of said further protein is lower than the level of casein. More preferably, the amount of said further protein is lower than both the level of casein and anti-coagulating protein. If present, said further protein, may be heat-sterilised together with the casein in step a), or together with the anti-coagulating protein in step b), or with both. Alternatively, the further protein, if present at all, may be separately heat-sterilised and added in step c) as a third component. Such a further protein serves a different purpose as influencing the coagulating status of the said mixture, for example, and if needed, to provide a source of amino acids which is complementary to the anti-coagulating and caseins, to improve organoleptic properties, or influence viscosity of the nutritional composition.

The invention also pertains to a heat-sterilised mixture of at least one lipid, casein and anti-coagulating proteins as a product obtainable by the process of claim 1. In an embodiment, said mixture is comprised by a composition, in particular a nutritional composition. Preferably, said composition is in liquid form (as an aqueous solution, emulsion or dispersion); however, it can also be advantageous to have said composition in dry form, e.g. in the form of a powder. The composition comprising said mixture, in particular a nutritional composition, is preferably heat-treated, in particular sterilised or pasteurised, or (ultra) filtrated.

### Product obtainable by the process of the invention

Furthermore, when said composition is in liquid form, the pH thereof preferably is in the range of 5-9, more preferably 6-8.

In a preferred embodiment, the heat-sterilisation of the liquid components in steps a) and b) is the final heat-treatment or sterilisation step before the composition is aseptically filled in at least one holder.

Preferably, the mixture of anti-coagulating and caseins constitutes, in liquid form, between 1 and 20% (w/v), preferably between 2 and 15% (w/v), more preferably between 4 and 12% (w/v), most preferably between 5 and 10% (w/v) of the composition, with or without further food components, preferably with further food components. More preferably, said composition is a nutritional composition, most preferably said composition is a liquid enteral nutritional composition.

The at least one lipid in the composition obtainable by the method of the present invention is preferably an oil and/or fat, and includes compounds comprising fatty acids as defined above. Thus, the lipid may comprise saturated fatty acids, mono unsaturated fatty acids (MU-FA), poly unsaturated fatty acids (PUFA), long-chain polyunsaturated fatty acids (1cPUFA) such as EPA and DHA, and may be provided by fish oil, vegetable oil and/or animal fats, more preferably fish oil and/or vegetable oil such as palm oil, canola oil and sunflower oil.

In a preferred embodiment, said lipid (preferably an oil and/or a fat) comprises between 1 and 30 wt% (w/v) of the composition obtainable according to the invention in liquid form, more preferably between 3 and 20 wt%, most preferably between 5 and 15 wt%.

In dry form, the contribution of the mixture of the anti-coagulating and caseins as a percentage of total weight of the composition depends on the presence of further food components as mentioned herein. The mixture of anti-coagulating and caseins can be used in a wide variety of food compositions, such as, but not limited to, enteral feeds, tube feeds or sip feeds, or used as an intermediate product for the manufacture of such feeds or food. Thus, where the dry form comprises further food components as mentioned herein, the amount of the mixture of anti-coagulating and casein constitutes between 10 and 90 wt% based on the total dry weight of the composition. Depending on the type of feed or food chosen, or its use as an intermediary product, the amount of the mixture of anti-coagulating and caseins varies between 8 and 90 wt%, 9 and 80 wt%, 10 and 70 wt%, 12 and 60 wt%, 13 and 50 wt%, 14 and 40 wt%, 15 and 30 wt% based on the total dry weight of the composition.

In a preferred embodiment, the mixture according to the present invention is part of a composition which comprises further food components. Such further food components are preferably present in food-grade quality, meaning they are heat-treated (e.g. pasteurised or sterilised) and/or (ultra) filtrated. The further food components typically comprise conventional food ingredients or constituents, such as digestible carbohydrates, fibres, vitamins minerals, single amino acids, dipeptides, tripeptides and oligopeptides. In a preferred embodiment, the further food components are selected from at least 1 wt% digestible carbohydrates and at least 0.1 wt% dietary fibres, relative to the total weight of said mixture of casein and anti-coagulating proteins comprised by said composition.

The relative amounts of proteins, digestible carbohydrates and fibres comprised by the composition according to the invention may vary according to the specific use and on whether it is a complete food or a food supplement. The protein portion thereof, in particular comprising said mixture according to the invention, in terms of energy (4 kcal/g) can e.g. be between 8 and 90 en.%. For a complete food said protein portion is preferably 8-40 en.%, more preferably 12-32 en.%, most preferably between 15 and 25 en.%. The lipid portion thereof in terms of energy (9 kcal/g) can e.g. be between 10 en.% and 92 en%. For a complete food said lipid portion is preferably 10-60 en.%, more preferably 15-50 en.%, most preferably between 20 and 40 en.%.

In another embodiment of the invention, a further protein, such as defined herein below, is encompassed by the mixture of at least one lipid, casein and anti-coagulating comprising nutritional composition. Preferably, the amount of said further protein in said composition is lower than the level of casein. More preferably, the amount of said further protein in said composition is lower than both the level of casein and anti-coagulating protein.

### Casein as coagulating protein

In general, coagulation means destabilisation or aggregation of proteins by decreasing their electric charge to that of the isoelectric point under the influence of acid and/or enzymes so that protein precipitates are formed. In the context of the process and product obtainable by the present invention, the casein preferably is micellar casein and/or a caseinate. Herein a casein is thus to be construed as including casein, caseinate, micellar casein, sodium caseinate, calcium caseinate, potassium caseinate and magnesium caseinate. Preferably, said casein is an intact casein, e.g. intact casein and/or intact caseinate. Herein, intact casein in particular means non-hydrolysed casein, i.e. having a degree of hydrolysis of less than 2%.

### Gastric Digestion Test

To investigate the coagulation properties of protein-comprising compositions, the Gastric Digestion Test according to Example 1 has been developed.

According to the present invention, a 6 wt% (w/v) protein solution with 5.8 wt% (w/v) of canola oil as a lipid source is incubated in the presence of artificial human saliva and artificial human gastric juice as defined below under physiologically suitable conditions (*i.e.* at 37°C under continuous stirring for 100 minutes), while controlling the pH such that the neutral starting pH of the protein solution (preferably a pH of 6) is reduced to a final pH of 2, after which the coagulate is size-fractioned and wet weight of the different fractions is determined. The wet weights of the different fractions represent a measure for the degree of coagulation.

The artificial human gastric juice as meant herein is a physiologically representative aqueous solution having 50 mM NaCl, 15 mM KCl, 1 mM CaCl₂.H₂O, 15 mM NaHCO₃, 0.014% (w/v) pepsin (e.g. porcine stomach, Sigma p7012), 0.019 % (w/v) lipase (*Rhizopus oryzae*, DF 15K Amano Pharmaceutical Co, Ltd Nagoya), at a pH of 4.0, of which 45 ml is used in the Gastric gestion Test. The artificial human saliva as meant herein is a physiologically representative aqueous solution having 100 mM NaCl, 30 mM KCl, 2 mM CaCl₂.H₂O, 0.065% (w/v) amylase (Sigma A 6211) having a pH of 6.3.

After incubation, the composition is divided in four fractions of different particle sises using a sieve. These four fractions are characterised as a) having a particle size of 0.25 mm or less, b) a particle size of between 0.25 and 1 mm, c) a particle size of between 1 and 2 mm and d) a particle size of larger than 2 mm. Especially coagulates having a size larger than 1 mm, or larger than 2 mm, are believed to play a role in delay of gastric emptying. However, reducing the amount of total coagulate, meaning herein all particles with a size larger than 0.25 mm, is believed to facilitate peptic digestion. The amount of the individual wet weight fractions is determined by weighing each individual sieve with coagulate on it and subtracting the weight of the respective sieve.

### Reduction of coagulation by anti-coagulating proteins

It has now been surprisingly found that the mixture of at least one lipid, casein and anti-coagulating proteins obtainable by the method of the present invention has reduced coagulation properties under the prevailing conditions of the upper gastro-intestinal tract, in particular the acidic conditions of the stomach, compared to the same mixture which is obtained by first mixing and subsequently heat-sterilising both proteins and the at least one lipid.

It is to be understood that the reduction on coagulation of casein in the stomach means that the addition of an anti-coagulating protein to a casein in the presence of at least one lipid yields a synergistic effect on reduction of coagulation beyond what is expected arithmetically.

Anti-coagulation thus means herein that a protein has the effect of reducing the coagulation of the casein with which it is combined in the presence of at least one lipid. This reduction effect on coagulation can be further characterised by the use of the herein described physiologically relevant Gastric Digestion Test. In a preferred embodiment, the anti-coagulating protein is characterised according to this digestion test, and qualifies as a protein which, when subjected to the method according to the present invention, reduces the size of coagulate particles in a synergistic manner. It preferably reduces the average size of coagulate particles of coagulating protein with a diameter of 0.25 mm or more in a detectable manner, compared to a processing method wherein the anti-coagulating protein is not absent during heat-sterilisation. Preferably, this reduction in the amount of particles with a size of 0.25 mm or more is at least 10 wt%. More preferably, the anti-coagulating protein reduces the amount of coagulate with particles of 1 mm or more in a detectable manner or by at least 10 wt%.

The reducing effect on coagulation is preferably determined using the Gastric Digestion Test with a 6% (w/v) solution of sodium caseinate and pea as anti-coagulating protein in the presence of 5.8% (w/v) canola oil as lipid source. Preferably, a weight ratio of 70:30 or 60:40 of sodium caseinate to anti-coagulating protein is used.

Anti-coagulating proteins are preferably selected such so as to provide an amino acid profile commensurate to the nutritional requirements of humans. In particular the anti-coagulating protein is selected to comply with the WHO amino acid profile recommendations for complete nutrition (see: WHO technical report series no. 935 - Protein and amino acid requirements in human nutrition: report of a joint FAO/WHO/UNU expert consultation, 2007). "Vegetal" relates to protein from plant origin, such as, for instance originating from vegetables such as carrot, pea, chickpea, green pea, cowpea, field pea, kidney bean, lupine, rice, soy, lentil, canola, hemp, zein, maize, corn, barley, flax, linseed, and wheat. Equivalent wording may be used, such as "vegetable", or "plant-derived". In particular, the vegetal protein is derived from leguminous plants (Fabaceae family).

The anti-coagulating protein is selected from pea and soy protein.

The anti-coagulating proteins used in the composition of the invention are preferably intact, i.e. non-hydrolysed.

### Specific anti-coagulating proteins

For the purpose of the present invention, pea protein, preferably intact pea protein, is a anti-coagulating protein. Preferably, pea protein is included in said mixture in an effective amount such that coagulation of said casein is reduced in the stomach of a subject.

Pea protein can be used effectively as anti-coagulating protein according to the process of the invention and composition obtainable thereby in a wide range of casein to anti-coagulating protein weight ratios as indicated, between 10:1 and 1:1, or between 4:1 and 1:1, or between 3:1 and 1:1, or between 10:1 and 1.5:1, or between 4:1 and 1.5:1, or between 3:1 and 1.5:1.

Pea protein is relatively inexpensive (on average, pea protein may cost about half the price of caseinates) and as it is added to the nutritional composition it increases the protein content while keeping costs quite low. Pea protein is generally tolerated well by most people, it is lactose-free and is not a common allergen. Pea protein is quite high in cysteine content and can therefore compensate the inadequate amount of cysteine in caseins. Furthermore, pea protein is quite high in arginine compared to casein, which is required for muscle metabolism and which facilitates the intake of body mass while reducing body fat; and it is quite high in lysine, when compared to the other vegetable proteins derived from cereals / grains, which is needed to build protein muscle and assist in the maintenance of lean body mass.

Several pea sources are readily available to the skilled person, for example, from Roquette (Lestrem, France) which markets a pea isolate obtained from yellow pea *(Pisum sativum*), and from Cosucra Groupe Warcoing (Warcoing, Belgium). Other pea protein sources may originate from green pea *(Pisum sativum)*, cowpea *(Vigna unguiculata)*, chickpea *(Cicerarietinum)*, and field pea *(Pisum arvense).*

In one embodiment according to the invention, the pea protein is substantially in intact form or non-hydrolysed. In another embodiment according to the invention, the pea protein is fermented pea protein or is pea protein hydrolysate.

In the context of this invention, a "non-hydrolysed" protein is equivalent to an "intact" protein, meaning that the protein has not been subjected to a hydrolysis process. However, minor amounts of hydrolysed proteins may be present in the source of non-hydrolysed proteins. In this context, "minor" should be understood as an amount of 10 weight% or less. The term "about" should be interpreted as a deviation of plus or minus 10 % of the given value.

For the purpose of the present invention, soy protein, preferably intact soy protein, is another anti-coagulating protein. Preferably, soy protein is included in said mixture in an effective amount such that coagulation of said casein is reduced in the stomach of a subject. Soy protein can be used in the process of the invention and included in the composition obtainable thereby in a wide range of the ratios as indicated herein, ranging between 10:1 and 1:1. The preferred ratio of casein to soy protein is such that moderate levels (e.g. between 4:1 and 1:1, or between 3:1 and 1:1, or between 10:1 and 1.5:1, or between 4:1 and 1.5:1, or between 3:1 and 1.5:1) of soy protein provide the most pronounced anti-coagulating effect. In a preferred embodiment, the anti-coagulating protein consists of soy protein, or consists of a combination with pea.

Soy protein is a vegetable protein that contains the essential amino acids in a relatively high proportion for human health. Several soy sources are readily available to the skilled person, for example, from The Solae Company (St. Louis, MO, USA).

In one embodiment according to the process or product of the invention, the soy protein is substantially in intact form or non-hydrolysed.

In another embodiment according to the process or product of the invention, the soy protein is fermented soy protein, or soy protein hydrolysate.

### Mixture of casein and anti-coagulating protein

The mixture of anti-coagulating and caseins according to the present invention in all its aspects (*i.e*. obtained by the process of the invention, encompassed by the composition obtainable by the process of the invention, has a weight ratio of casein to anti-coagulating protein of between 10:1 and 1:1.

It is within the capabilities of the skilled person to establish what exact ratio is preferred to reduce the gastric coagulation properties of a given casein by selecting an amount and type of anti-coagulating protein using the Gastric Digestion Test as meant herein.

Preferably, the composition obtainable by the process of the invention is a liquid composition which comprises said mixture of casein and anti-coagulating protein obtainable by step c) and at least one lipid.

The amount of the protein mixture obtainable by step c) as included in the liquid composition comprises between 1 and 20% (w/v) of said liquid composition, preferably between 2 and 15% (w/v), more preferably between 4 and 12% (w/v), most preferably between 5 and 10% (w/v) of said liquid composition. More specifically, the amount of the protein mixture as obtainable by step c) of the invention comprises 1, 2, 3, 4, 5, 6, 7, 8, 9, 10, 11, 12, 13, 14, 15, 16, 17, 18, 19 or 20% (w/v) of said liquid composition. Most preferably, the protein content of the liquid composition 6% (w/v).

### Applications

Tube feeding is given to provide nutrition to patients which cannot obtain nutrition by swallowing, using a device such as a nasogastric feeding tube or a naso jejunal feeding tube, or by using a percutaneous endoscopic gastrostomy (PEG) or PEG - jejuno-feeding system. In the context of this application, the state of being fed by nutritional supplements and/or a by a feeding tube is called enteral feeding, comprising all of the abovementioned tube feeding systems, and the nutrition used in the feeding by nutritional supplements and/or a by a feeding tube is called enteral nutrition.

In a preferred embodiment, said composition comprising at least one lipid and said mixture of the invention is a nutritional composition which is administered to humans, preferably to humans that benefit from receiving easily digestible nutrition. Preferably, said administering involves oral administration, by eating or drinking, preferably enterally by tube feeding, of the composition with the mixture of the present invention.

Another category of subjects that can benefit from the present method are infants. Thus in one embodiment according to the present invention the nutritional composition is an infant formula, a follow-on formula and/or a toddler formula. In one embodiment according to the present invention the nutritional composition is in a suitable form for administration to a baby, an infant and/or a toddler.

Preferably, the mixture of the invention and the at least one lipid comprised by a (nutritional) composition can be used to partially or fully replace, or supplement, the typical daily intake by a person of caseins which are produced by conventional methods, i.e. methods not including the combination of separately heat-sterilising casein and anti-coagulating proteins in steps a) and b), with the mixing thereof in step c) according to the present invention.

The composition according to the invention comprising said protein mixture and at least one lipid preferably has the form of a complete food, *i.e.* it can meet all nutritional needs of the user. As such, the composition is preferably in the form of a liquid, more preferably a liquid enteral composition according to the invention, and as such preferably contains 1000 to 2500 kcal per daily dosage. Depending on the condition of the patient, a daily dose is about 25 to 35 kcal/kg body weight/day. Therefore, a typical daily dose for a 70 kg person contains about 2000 kcal. The complete food can be in the form of multiple dosage units, e.g. from 8 (250 ml/unit) to 2 units (1 1/unit) per day for an energy supply of 2000 kcal/day using a liquid enteral composition according to the invention of 1.0 kcal/ml. Preferably, the composition is adapted for tube feeding.

Although a complete food composition of the invention may, in addition to the protein mixture of the invention comprising at least one casein and at least one anti-coagulating protein, contain further proteins sources, it is preferred that the proteins mixture of the invention is the only protein source. The protein mixture of the invention preferably contributes to between 5 and 40 en.%, more preferably between 8 and 32 en.%, most preferably between 15 and 25 en.% of the complete food. The daily dosage of the protein mixture of the invention is preferably between 3 kcal and 10 kcal per kg bodyweight, more preferably between 4 and 8 kcal per kg bodyweight, or preferably between 0.75 and 2.5 g protein mixture, preferably between 1 and 2 g per patient per day, as part of a complete food.

Preferably, the composition is packaged, stored and provided in a container such as plastic bag or a pouch or the like. A variety of such containers is known, for example 500 ml, 1000 ml, and 1500 ml containers are known in the art. It should be noted that any suitable container can be used to package, store and provide the nutritional composition according to the invention.

In one embodiment of the present invention, the composition is provided in a ready to use liquid form and does not require reconstitution or mixing prior to use. The composition according to the invention can be tube fed or administered orally. For example, the composition according to the invention can be provided in a can, on spike, and hang bag. However, a composition may be provided to a person in need thereof in powder form, suitable for reconstitution using an aqueous solution or water such that the composition according to the invention is produced. Thus in one embodiment of the present invention, the present composition is in the form of a powder, accompanied with instructions to dissolve or reconstitute in an aqueous composition or water to arrive at the liquid enteral composition according to the present invention. In one embodiment of the present invention, the present liquid enteral composition may thus be obtained by dissolving or reconstituting a powder, preferably in an aqueous composition, in particular water.

The invention will now be further elucidated by several examples, without being limited thereto or thereby.

### FIGURES

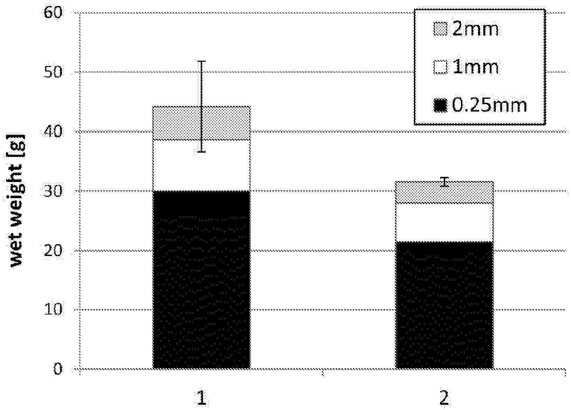

Figure 1 shows the absolute wet weight of coagulates between 0.25 mm and 1 mm (lower part, black bars), between 1mm and 2 mm (middle part, open bars) and larger than 2 mm (upper part, grey bars), after 100 minutes of gastric digestion of protein mixtures of sodium caseinate with pea protein and canola oil as lipid source. 1: Na-cas:pea = 60:40 ratio; heat-sterilised as a single protein mixture, 2: same ratio, casein and pea mixed after separate heat-sterilisation.

### EXAMPLES

### Example 1

### Preparation of protein/lipid mixtures

Commercially available canola oil, sodium caseinate with a protein content of 88 wt%, pea protein with a protein content of 78 wt% were used as starting materials for obtaining the solutions as prepared.

Three different dispersions (1, 2a and 2b) were prepared by mixing the protein sources into demineralised water. Dispersion 1 was obtained by mixing sodium caseinate and pea protein (weight ratio of 60:40 sodium caseinate to pea protein) and canola oil. Dispersion 2a contained only sodium caseinate, dispersion 2b contained pea protein and canola oil. For all dispersions, the pH was then adjusted using potassium hydroxide to reach pH 8.0. After pH adjustment, the three different mixtures were heated for 30 seconds at 85°C, which was followed by a homogenisation step. After pasteurisation, the pH was again adjusted to pH 8.0 and the dry matter content was adjusted to come to a final protein content of all dispersion of 6 wt % (w/v) and a final fat content of 5.8 wt% (w/v). Finally, all products were filled in 200 ml glass bottles which were retort sterilised for 16 minutes at 121.5°C.

The sterilised dispersion 1 was subsequently used in the Gastric Digestion Test. Dispersions 2a and 2b were mixed after sterilisation to obtain a 60:40 sodium caseinate to pea protein ratio and subsequently subjected to the Gastric Digestion Test.

### Gastric Digestion Test

Stomach digestion and coagulation of the prepared samples were mimicked over 100 minutes in a computer-controlled substrate pump setup (Multifermentor fed-batch; DASGIP AG, Juelich, Germany) at 37°C upon continuous stirring.

For each experiment, 150 ml of the obtained protein/lipid solution was used as the starting volume. Per experiment, a total of 45 ml of artificial gastric juice (50 mM NaCl, 15 mM KCl, 1 mM CaCl₂.H₂O, 15 mM NaHCO₃, 0.014 % (w/v) pepsin (porcine stomach, sigma p7012), 0.019 % (w/v) lipase (*Rhizopus oryzae*, DF 15K Amano Pharmaceutical Co, Ltd Nagoya); pH 4.0) was added. The gastric juice was added in two steps with different flow rates. In the first two minutes, a flow rate of 225 ml/h was used. For the rest of the experiment the flow rate was 23 ml/h. In addition, in the first 60 minutes of the experiment a total of 30 ml of artificial saliva (100 mM NaCl, 30 mM KCl, 2 mM CaCl₂.2H₂O, 15 mM NaHCO₃, 0.065 % (w/v) α-amylase (Sigma A 6211); pH 6.3) was added continuously to the solution at a constant rate.

The pH was decreased over 100 minutes from a pH of 6.6 at start to a final pH of 2.0 (pH at start = 6.6, pH at 8 minutes = 5.0, at 15 minutes = 4.0, at 42 minutes = 3.0, at 100 minutes = 2.0) by the addition of 1 M HCl upon continuous mixing. If necessary, acidification was automatically corrected by the addition of an alkaline solution (1 M NaHCO₃, 3 M NaOH).

### Determination of Coagulate

After gastric digestion, each sample was poured over metal sieves to yield fractions with particle sizes of a) larger than 2 mm, b) below 2 mm and above 1 mm, c) below 1 mm and above 0.25 mm and d) below the limit of 0.25 mm. In short, the wet weight fractions were determined by weighing each individual respective sieve with coagulate on it and subtracting the weight of each sieve.

### Control results

As a control, the coagulation properties upon gastric digestion for a solution with sodium caseinate as the only protein component at a concentration of 6 wt% (w/v) and canola oil as a lipid source at a concentration of 5.8 wt% (w/v) were investigated. This control sample showed high coagulate wet weight levels after subjecting it to the below mentioned Gastric Digestion Test (data not shown).

### Sample results

After subjecting samples to the treatment as mentioned under "Preparation of protein/lipid mixtures", pea protein which was sterilised in a single solution together with sodium caseinate in a 60:40 weight ratio and canola oil gave a total amount of coagulate of about 45 wt% based on wet weight (FIG 1, sample 1). However, when the same mixture of pea and sodium caseinate and canola oil was obtained by first sterilising and then mixing said proteins, the coagulate amounts of each individualised fraction and total coagulate wet weight were significantly reduced (FIG 1, sample 2).

### Test for anti-coagulating proteins

A protein under investigation is considered to be anti-coagulating according to the present invention, if in the experiment above (the Gastric Digestion Test of Example 1) a mixture of the heat-sterilised protein and a separately heat-sterilised casein produces an amount of wet weight coagulate fraction larger than 0.25 mm which is at least 10% less than the amount produced by the same protein mixture prepared by first mixing the two different protein components followed by heat-sterilising the mixed protein components.

## Claims

1. A process of producing a composition comprising at least one lipid and a mixture of two different proteins, of which at least one is a casein and at least one is an anti-coagulating protein, comprising the steps of:
a) heat-sterilising a first liquid component which comprises said casein in an amount of at least 85wt% of the total protein content of the first component, and wherein said first liquid component comprises less than 5wt% anti-coagulating protein based on the total protein content of the first liquid component, and
b) heat-sterilising a second liquid component comprising said anti-coagulating protein, wherein said anti-coagulating protein is selected from pea protein and soybean protein; and wherein said second liquid component comprises less than 0.1 wt% of casein based on the total protein content of the second liquid component, and
c) mixing said first component with said second component to obtain a mixture of said proteins, wherein said first and/or said second liquid component comprises said lipid, preferably at least said second liquid component comprises said lipid, and wherein said mixture has a weight ratio of said casein to said anti-coagulating protein of between 10:1 and 1:1.

2. The process according to claim 1, wherein said casein is selected from the group consisting of micellar casein, non-micellar casein, sodium caseinate, calcium caseinate, potassium caseinate and magnesium caseinate.

3. The process according to any one of claims 1-2, further comprising, after mixing in step c), the step of drying said mixture to obtain a powder; or comprising said mixture by a liquid and filling said liquid into a holder, preferably aseptically filled into a holder.

4. The process according to any one of claims 1-2, wherein said mixture of step c) is comprised by a liquid composition and said mixture is present in an amount of between 1 and 20% (w/v), preferably between 2 and 15% (w/v), more preferably between 4 and 12% (w/v), most preferably between 5 and 10% (w/v) in said liquid composition.

5. A dry or liquid composition obtainable by the process according to any one of claims 1-4.

6. A composition according to claim 5, comprising a mixture of casein and anti-coagulating protein which, in the Gastric Digestion Test of Example 1, produces an amount of wet weight coagulate fraction larger than 0.25 mm which is at least 10% less than the amount produced by the same protein mixture prepared by first mixing the two different protein components followed by heat-sterilising the mixed protein components.

7. A non-therapeutic process for reducing coagulation in the upper gastrointestinal tract in a person, comprising administering or consuming the composition according to any one of claims 5 and 6.

8. Non-therapeutic use of the composition according to any one of claims 5 and 6 in the reduction of coagulation in the upper gastrointestinal tract in a person.

## Patentansprüche

1. Ein Verfahren zur Herstellung einer Zusammensetzung, umfassend mindestens ein Lipid und eine Mischung von zwei unterschiedlichen Proteinen, von denen mindestens eins ein Casein ist und mindestens eins ein gerinnungshemmendes Protein ist, umfassend die Schritte:
a) thermische Sterilisierung einer ersten flüssigen Komponente, die das Casein in einer Menge von mindestens 85 Gew.-% des vollständigen Proteingehalts der ersten Komponente umfasst, wobei diese erste flüssige Komponente weniger als 5 Gew.-% gerinnungshemmendes Protein, bezogen auf den vollständigen Proteingehalt der ersten flüssigen Komponente, umfasst,
b) thermische Sterilisierung einer zweiten flüssigen Komponente, umfassend das gerinnungshemmende Protein, wobei dieses gerinnungshemmende Protein ausgewählt ist aus Erbsenprotein und Sojabohnenprotein, und wobei diese zweite flüssige Komponente weniger als 0,1 Gew.-% Casein, bezogen auf den vollständigen Proteingehalt der zweiten flüssigen Komponente, umfasst, und
c) Mischen der ersten Komponente mit der zweiten Komponente, um eine Mischung der Proteine zu erhalten, wobei die erste und/oder die zweite flüssige Komponente das Lipid umfasst/umfassen und vorzugsweise mindestens diese zweite flüssige Komponente das Lipid umfasst, und wobei diese Mischung ein Gewichtsverhältnis von dem Casein zu dem gerinnungshemmenden Protein von zwischen 10 : 1 und 1 : 1 besitzt.

2. Das Verfahren gemäß Anspruch 1, wobei das Casein ausgewählt ist aus der Gruppe, bestehend aus micellarem Casein, nicht-micellarem Casein, Natriumcaseinat, Calciumcaseinat, Kaliumcaseinat und Magnesiumcaseinat.

3. Das Verfahren gemäß einem der Ansprüche 1-2, das nach dem Mischen in Schritt c) ferner den Schritt des Trocknens der Mischung umfasst, um ein Pulver zu erhalten; oder umfassend die Mischung als eine Flüssigkeit und diese Flüssigkeit in einen Halter füllend, vorzugsweise aseptisch in einen Halter füllend.

4. Das Verfahren gemäß einem der Ansprüche 1-2, wobei die Mischung aus Schritt c) umfasst ist durch eine flüssige Zusammensetzung und diese Mischung in einer Menge von zwischen 1 und 20 % (w/v), vorzugsweise zwischen 2 und 15 % (w/v), noch bevorzugter zwischen 4 und 12 % (w/v) und am meisten bevorzugt zwischen 5 und 10 % (w/v) in der flüssigen Zusammensetzung vorliegt.

5. Eine trockene oder flüssige Zusammensetzung, erhältlich durch das Verfahren gemäß einem der Ansprüche 1-4.

6. Eine Zusammensetzung gemäß Anspruch 5, umfassend eine Mischung von Casein und gerinnungshemmendem Protein, die im Magenverdauungstest aus Beispiel 1 eine Menge der Nassgewichtsgerinnungsfraktion von größer als 0,25 mm erzeugt, welche mindestens 10 % geringer ist als die Menge, die durch die gleiche Proteinmischung, die durch zuerst Mischen der beiden unterschiedlichen Proteinkomponenten und anschließende thermische Sterilisierung der gemischten Proteinkomponenten hergestellt ist, erzeugt wird.

7. Ein nicht-therapeutisches Verfahren zum Verringern der Gerinnung im oberen Gastrointestinaltrakt in einer Person, umfassend die Verabreichung oder Einnahme der Zusammensetzung gemäß einem der Ansprüche 5 und 6.

8. Nicht-therapeutische Verwendung der Zusammensetzung gemäß einem der Ansprüche 5 und 6 zum Verringern der Gerinnung im oberen Gastrointestinaltrakt in einer Person.

## Revendications

1. Procédé de production d'une composition comprenant au moins un lipide et un mélange de deux protéines différentes, dont au moins une est une caséine et au moins une est une protéine anti-coagulante, comprenant les étapes :
a) stériliser à la chaleur un premier composant liquide qui comprend ladite caséine en une quantité d'au moins 85 % en poids de la teneur totale en protéines du premier composant, et dans lequel ledit premier composant liquide comprend moins de 5 % en poids de protéine anti-coagulante par rapport à la teneur totale en protéines du premier composant liquide, et
b) stériliser par la chaleur un second composant liquide comprenant ladite protéine anticoagulante, dans lequel ladite protéine anticoagulante est choisie parmi les protéines de pois et les protéines de soja ; et dans lequel ledit second composant liquide comprend moins de 0,1 % en poids de caséine par rapport à la teneur totale en protéines du second composant liquide, et
c) mélanger ledit premier composant avec ledit second composant pour obtenir un mélange desdites protéines, dans lequel ledit premier et/ou ledit second composant liquide comprend ledit lipide, de préférence au moins ledit second composant liquide comprend ledit lipide, et dans lequel ledit mélange a un rapport pondéral de ladite caséine à ladite protéine anti-coagulante compris entre 10:1 et 1:1.

2. Procédé selon la revendication 1, dans lequel ladite caséine est choisie dans le groupe constitué par la caséine micellaire, la caséine non micellaire, le caséinate de sodium, le caséinate de calcium, le caséinate de potassium et le caséinate de magnésium.

3. Procédé selon l'une quelconque des revendications 1 à 2, comprenant en outre, après le mélange de l'étape c), l'étape de séchage dudit mélange pour obtenir une poudre ; ou comprenant ledit mélange par un liquide et remplissant ledit liquide dans un support, de préférence rempli de manière aseptique dans un support.

4. Procédé selon l'une quelconque des revendications 1 et 2, dans lequel ledit mélange de l'étape c) est comprise dans une composition liquide et ledit mélange est présent en une quantité comprise entre 1 et 20% (p/v), de préférence entre 2 et 15% (p/v), plus préférablement entre 4 et 12% (p/v), le plus préférablement entre 5 et 10% (p/v) dans ladite composition liquide.

5. Composition sèche ou liquide pouvant être obtenue par le procédé selon l'une quelconque des revendications 1 à 4.

6. Composition selon la revendication 5, comprenant un mélange de caséine et de protéine anti-coagulante qui, dans le test de digestion gastrique de l'exemple 1, produit une quantité de fraction coagulée en poids humide supérieure à 0,25 mm qui est inférieure d'au moins 10% à la quantité produite par le même mélange de protéines préparé en mélangeant d'abord les deux composants protéiques différents puis en stérilisant à la chaleur les composants protéiques mélangés.

7. Procédé non-thérapeutique pour réduire la coagulation dans le tractus gastro-intestinal supérieur chez une personne, comprenant l'administration ou la consommation de la composition selon l'une quelconque des revendications 5 et 6.

8. Utilisation non-thérapeutique de la composition selon l'une quelconque des revendications 5 et 6 dans la réduction de la coagulation dans le tractus gastro-intestinal supérieur chez une personne.
